# EUROPEAN PATENT APPLICATION

(11) **EP 4 674 467 A1**
(43) Date of publication of application: **07.01.2026**
(21) Application number: 24185752.3
(22) Date of filing: 01.07.2024
(51) Int. Cl.: A61N 1/04, A61N 1/36

(54) **DEVICE FOR TRANSCUTANEOUS AURICULAR NERVE STIMULATION**

(71) Applicant: Aurimod GmbH, 1030 Wien (AT)
(72) Inventor: LE, Van Hoang, 1030 Wien (AT); ZEINER, Klaus, 1030 Wien (AT)
(74) Representative: SONN Patentanwälte GmbH & Co KG

(57) **Abstract**

A device for transcutaneous auricular nerve stimulation, comprising:
a first stimulation assembly (1) comprising a first housing configured to be positioned over a user's first ear,
a second stimulation assembly (2) comprising a second housing configured to be positioned over a user's second ear,
the first and the second housing each comprising an electrode arrangement configured to contact a skin surface in proximity to the user's first and second ear, respectively,
a holding structure (3) coupled to the first stimulation assembly (1) and to the second stimulation assembly (2) and configured to be worn on or around the user's head,
a stimulation circuit (13) electrically coupled to the electrode arrangement and configured to generate electrical stimulation signals to be delivered by the electrode arrangement,
wherein the electrical stimulation signals delivered by the electrode arrangement are configured to stimulate nerves in the region of the user's first ear and the user's second ears, respectively.

## Description

Conventional transcutaneous auricular stimulators have been developed for various applications, such as opioid withdrawal, migraine, etc. These devices typically involve applying electrical stimuli to the outer and/or inner ear using dry or wet surface electrodes. However, these existing solutions often suffer from limited user-friendliness and require bulky handheld devices that can be cumbersome and unappealing for daily use.

While transcutaneous stimulators using surface electrodes offer convenience and ease of use, percutaneous stimulators with needle electrodes provide more robust stimulation with smaller devices. However, the continuous wearing of these devices for 24 hours and regular visits to the doctor for device application may not be suitable or necessary for all patients.

Conventional transcutaneous auricular nerve stimulators often require separate electrode placement, attachment, and management, making the setup process cumbersome and time-consuming for users. This can lead to decreased compliance and limited adoption of these devices as part of daily routines or therapeutic regimens.

Traditional transcutaneous auricular nerve stimulators typically involve separate controlling devices that are often large, heavy, and unappealing for daily use. These controlling devices can be inconvenient to carry around and may discourage users from incorporating the therapy into their everyday lives.

The design of conventional transcutaneous auricular nerve stimulators does not allow for concurrent sound delivery, which may be beneficial for specific means, thus limiting their potential applications and user experience. In-ear applications have the drawback of having a restricted placement of electrodes, which might not be able to stimulate numerous nerves located around the ear. Additionally, their battery capacity tends to be smaller, leading to more frequent charging compared to over-ear headphones.

Traditional transcutaneous auricular nerve stimulators are often not designed to be portable or integrated into compact, all-in-one devices, making it challenging for users to utilize the therapy in various settings and during daily activities. This lack of portability can hinder therapy adherence and limit accessibility for some individuals.

Conventional transcutaneous auricular nerve stimulators may not provide real-time impedance measurement, making it difficult for users and healthcare professionals to assess electrode contact quality, safety, and effectiveness. Accurate impedance monitoring is crucial in ensuring optimal stimulation performance and tailoring therapy parameters based on individual needs.

The high cost and limited availability of traditional transcutaneous auricular nerve stimulators can hinder widespread adoption and utilization. By incorporating these devices into more accessible and versatile systems, such as over-ear headphones, a broader audience could benefit from the therapy.

Current auricular nerve stimulators are specialized for a specific region and/or nerve. However, should multiple nerves require stimulation, the use of various specialized devices becomes necessary, which can get very expensive or not suitable.

Therefore, the instant invention aims to address the limitations and drawbacks of conventional transcutaneous auricular nerve stimulators by providing a portable, user-friendly, and versatile device that integrates electrical stimulation in a compact, non-invasive design. In particular, an object of the invention is to offer a convenient and accessible solution for individuals seeking auricular nerve stimulation therapy, i.e., stimulation of peripheral nerves like the lesser occipital nerve, the auriculotemporal nerve, the auricular branch of the vagus nerve, the glossopharyngeal nerve, the facial nerve, or the greater auricular nerve, for various neuromodulation applications, such as pain management, the treatment of inflammatory, neurological or psychiatric diseases, stress reduction or sleep improvement.

In order to solve this and other objects, the invention provides a device for transcutaneous auricular nerve stimulation, comprising:
a first stimulation assembly comprising a first housing configured to be positioned over a user's first ear,
a second stimulation assembly comprising a second housing configured to be positioned over a user's second ear,
the first and the second housing each comprising an electrode arrangement configured to contact a skin surface in proximity to the user's first and second ear, respectively,
a holding structure coupled to the first stimulation assembly and to the second stimulation assembly and configured to be worn on or around the user's head,
a stimulation circuit electrically coupled to the electrode arrangement and configured to generate electrical stimulation signals to be delivered by the electrode arrangement,
wherein the electrical stimulation signals delivered by the electrode arrangement are configured to stimulate nerves in the region of the user's first ear and the user's second ears, respectively.

Thus, the invention provides an over-ear headphone design, wherein the first and second stimulation assemblies correspond to the left and right earpieces or earcups of the over-ear headphones, respectively. The housings of these assemblies are designed to fit over the user's ears, enclosing them and providing a secure, stable fit. The over-ear design allows for larger housings compared to in-ear headphones, providing more space for the integration of stimulation components and larger battery capacity.

The electrode arrangements are each integrated into the earpiece, positioned to make contact with the skin around the user's ears when the headphones are worn. The over-ear design provides a larger contact area between the electrodes and the skin, enabling the stimulation of a wider range of auricular nerve branches and zones. In this way, the invention enables a targeted stimulation to various auricular nerve branches or zones, such as the facial nerve, vagus nerve, occipital nerve, greater auricular nerve, auriculotemporal nerve, and glossopharyngeal nerve. The electrode arrangement can be configured to deliver electrical impulses to different sets of electrodes, allowing for the selective stimulation of specific nerve regions. This targeted approach enables the invention to address a wide range of therapeutic goals and conditions related to neuromodulation, such as pain management, the treatment of inflammatory, neurologic or psychiatric diseases, stress reduction or sleep improvement.

The secure fit of the over-ear headphones ensures consistent and reliable contact between the electrodes and the skin, enhancing stimulation effectiveness.

The holding structure corresponds to the headband of the over-ear headphones, connecting the left and right earpieces. The headband is designed to be worn over the top of the user's head or around the back of the head, depending on user preference and comfort. The holding structure provides stability and support for the earpieces, ensuring they remain securely positioned over the ears during use.

The over-ear headphone design allows the device to be easily and comfortably worn on or around the user's head. The headband and earpieces may preferably be adjustable to accommodate different head sizes and shapes, ensuring a comfortable and secure fit for a wide range of users. The over-ear design is familiar to many users, making the device more approachable and easier to incorporate into daily life compared to specialized medical devices. Furthermore, the integration of stimulation electrodes within over-ear headphones eliminates the need for separate electrode placement and attachment, streamlining the setup process and improving overall user experience.

It should be noted that the present invention is not limited to embodiments wherein the over-ear headphone design necessarily includes audio output capabilities. The term "over-ear headphone design" is used primarily to describe the shape, form factor, and configuration of the device, which includes earpieces that fit over the user's ears and a connecting headband. This design choice is independent of the device's ability to produce sound. The invention may be implemented in various embodiments, some of which may include audio output functionality, while others may focus solely on the delivery of transcutaneous auricular nerve stimulation without any audio capabilities.

The present invention further comprises a stimulation circuit that is electrically coupled to the electrode arrangement and configured to generate electrical stimulation signals to be delivered by the electrode arrangement. The stimulation circuit is designed to produce electrical impulses with specific characteristics, such as waveform, frequency, pulse width, amplitude, and duration, which are suitable for stimulating the targeted auricular nerves in the region of the user's first and second ears.

In one embodiment, the stimulation circuit includes a microcontroller or processor that executes firmware or software instructions to generate the desired stimulation signals. The microcontroller is coupled to a digital-to-analog converter (DAC) that converts the digital signals generated by the microcontroller into analog waveforms. The analog waveforms are then amplified by a power amplifier to provide the necessary current and voltage levels for effective nerve stimulation.

In another embodiment, the stimulation circuit may include a waveform generator, such as a dedicated integrated circuit or a programmable logic device, that produces the desired stimulation waveforms. The waveform generator can be configured to output various waveform shapes, such as square waves, sine waves, or triangular waves, with adjustable frequency, pulse width, amplitude, and duty cycle. The output of the waveform generator is then conditioned by signal conditioning circuitry, which may include filters, amplifiers, and safety limiters, before being delivered to the electrode arrangement.

The stimulation circuit is powered by a battery or other power source, such as a rechargeable lithium-ion battery, which provides sufficient energy to generate the electrical stimulation signals for the duration of the intended use. The battery may be housed within one of the first and the second housings or within a separate compartment in the holding structure, depending on the design constraints and power requirements of the device.

According to a preferred embodiment of the invention, the electrode arrangement comprises at least one, preferably at least two electrodes arranged in a skin-contacting region of the first and second housing, respectively. The skin-contacting region is configured to surround the user's first and second ear, respectively. By positioning the electrodes around the ear, the device can stimulate various regions of the auricular nervous system, such as the vagus nerve, trigeminal nerve, or the auriculotemporal nerve. With at least two electrodes surrounding each ear, the device can create localized electrical fields that specifically target the desired nerve regions while minimizing the stimulation of unintended areas.

The electrode arrangement surrounding the user's ears also provides a larger contact area between the electrodes and the skin, which offers several technical benefits. The increased contact area allows for a more efficient current delivery, as the electrical resistance between the electrodes and the skin is reduced. Additionally, the larger contact area helps to distribute the stimulation current more evenly across the skin surface, minimizing the risk of localized discomfort or irritation.

The electrode arrangement, in a preferred embodiment, comprises a plurality of electrodes spaced apart in the circumferential direction of the skin-contacting region. This offers the ability to selectively stimulate different auricular nerve branches or zones by activating specific subsets of electrodes. By arranging the electrodes in a circumferential pattern around the ear, the device can target nerve fibers located in various regions of the auricular anatomy. Furthermore, the circumferentially spaced electrode arrangement enhances the spatial resolution and precision of the stimulation delivery. By increasing the number of electrodes and distributing them along the circumference of the skin-contacting region, the device can create more localized and focused electrical fields. This improved spatial resolution allows for the fine-tuning of the stimulation pattern to match the individual user's auricular nerve distribution and sensitivity. The device can selectively activate or deactivate specific electrodes to optimize the stimulation coverage and intensity for each user.

According to another preferred embodiment of the invention, the skin-contacting region comprises a resilient pad configured to contact a skin surface surrounding the user's first and second ear, respectively, wherein the electrodes are embedded into or arranged on the resilient pad. One technical effect of the resilient pad is its ability to conform to the contours and shape of each user's ear and the surrounding skin surface. The resilient material, such as a soft silicone or foam, allows the pad to adapt to the individual's ear anatomy, ensuring a snug and comfortable fit. This conformity promotes consistent and reliable contact between the electrodes and the skin, which is advantageous for effective stimulation delivery. The resilient nature of the pad helps to maintain this contact even during slight movements, minimizing the risk of electrode displacement or loss of contact. Furthermore, the conforming properties of the pad help to distribute the pressure evenly across the skin surface, reducing the likelihood of localized discomfort or irritation, even during extended periods of use.

Preferably, the resilient pad is removably attached to a base part of the first and the second housing, respectively, via a coupling mechanism, wherein the coupling mechanism preferably comprises either a magnetic coupling mechanism or a mechanical coupling mechanism, such as a snap-fit, hook-and-loop fastener, or other releasable fastening means, thereby achieving an increased modularity and adaptability of the device. By allowing the resilient pad to be easily detached and reattached, users can swap between different pad sizes, shapes, or materials to accommodate their individual ear anatomy and preferences. Moreover, the removable nature of the pad allows for the replacement of worn or damaged pads without the need to replace the entire device.

Another technical effect of the removable attachment of the resilient pad is the simplified maintenance and cleaning process. The ability to detach the pad from the housing base part allows users to thoroughly clean and sanitize the pad separately, ensuring optimal hygiene and reducing the risk of bacterial growth or skin irritation. The removable design also enables the inspection and replacement of individual components, such as the electrodes or the resilient material, without the need to disassemble the entire device. This modularity streamlines the maintenance process, reduces downtime, and prolongs the overall lifespan of the device. Additionally, the removable attachment feature allows for the storage or transportation of the pads separately from the main device, minimizing the risk of damage or contamination during non-use periods.

According to a preferred embodiment, the device of the invention further comprises a control unit configured to control a stimulation parameter of the electrical stimulation signals, such as the intensity, frequency, pulse width, and/or duration of the electrical stimulation signals. The control unit may provide a user interface that allows users to easily adjust and monitor the stimulation parameters. The interface may include visual displays, such as LED indicators or a digital screen, that provide real-time information about the current stimulation settings and the device's status. Users can interact with the control unit through controls, such as buttons, dials, or touch-sensitive surfaces, to modify the stimulation parameters according to their preferences or as directed by their healthcare provider. The control unit may also include memory functions that allow users to save and recall their preferred stimulation settings, facilitating consistent and convenient use of the device across multiple sessions. Additionally, the control unit can be programmed to deliver pre-set stimulation protocols or therapy regimens, which can be easily selected and initiated by the user.

When stimulating the auricular nerves using transcutaneous electrical stimulation, several key parameters are considered and adjusted to optimize the therapy. These parameters include the intensity (amplitude), frequency, pulse width, and duration of the stimulation signals. The selection and adjustment of these parameters depend on various factors, such as the targeted nerve branches, the desired physiological effects, and the individual user's tolerance and response to the stimulation.

### Intensity (Amplitude):

The intensity or amplitude of the stimulation refers to the strength of the electrical current delivered to the auricular nerves. It is typically measured in milliamperes (mA) and is adjusted to ensure that the stimulation is perceptible but not uncomfortable for the user. The optimal intensity level varies among individuals and is determined through a gradual ramping-up process, starting from a low intensity and increasing until the user feels a tingling or mild buzzing sensation. The intensity is usually set at a level just below the user's discomfort threshold to maximize the therapeutic effects while minimizing any unpleasant sensations. Typical intensity ranges for auricular nerve stimulation are between 0.5 mA to 5 mA, depending on the user's sensitivity and the electrode placement.

### Frequency:

The frequency of the stimulation refers to the number of electrical pulses delivered per second, measured in Hertz (Hz). The choice of stimulation frequency is based on the desired physiological effects and the specific nerve fibers being targeted.

### Pulse Width:

The pulse width refers to the duration of each electrical pulse, measured in microseconds (ps). It determines the amount of time the stimulation current is applied to the nerves during each pulse. Longer pulse widths can recruit more nerve fibers and produce stronger physiological responses, but they may also increase the likelihood of discomfort. Shorter pulse widths are generally more comfortable but may require higher intensities to achieve the desired effects. Typical pulse widths used in auricular nerve stimulation range from 50 µs to 500 µs, with 200-300 µs being commonly employed.

### Duration:

The duration of the stimulation refers to the length of each stimulation session or the overall treatment period. The optimal duration depends on the specific therapeutic goals and the user's response to the stimulation. Shorter sessions (10-60 minutes), sometimes repeated 3-4 times over one day, are often used for acute symptom relief, while longer sessions (30-120 minutes), often repeated continuously only with short pauses (30-60 minutes) in between stimulation sessions, may be employed for chronic conditions. The duration of the stimulation can be gradually decreased (weaning off) or increased over time to maintain the therapeutic effects, to prevent adaptation or habituation to the stimulation, or to account for a long-lasting therapeutic effect which builds-up over prolonged periods of stimulation (2-6 weeks or longer) and thus reduces the need for further stimulation therapy.

It should be noted that the electrical stimulation circuit and the control unit described in the present invention are not necessarily separate devices or entities, but can also be realized in an integrated manner. The terms "electrical stimulation circuit" and "control unit" may refer to different functionalities or modules within a single, unified circuitry.

In a preferred embodiment of the invention, the electrical stimulation circuit comprises a switching means electrically coupled to the plurality of electrodes in the electrode arrangement, the switching means configured to selectively connect the electrical stimulation circuit to at least one selected pair of electrodes out of the plurality of electrodes in the electrode arrangement based on switching instructions received from the control unit. The control unit may in such an embodiment be further configured to i) generate the switching instructions for the switching means based on a desired stimulation pattern and electrode configuration, and ii) transmit the switching instructions to the switching means to selectively connect the electrical stimulation circuit to the selected pairs of electrodes in the electrode arrangements, thereby enabling the targeted delivery of electrical stimulation signals to specific regions in proximity to the user's first and second ears.

By selectively activating different pairs of electrodes within the electrode arrangement the device can dynamically target specific auricular nerve branches or regions. By receiving switching instructions from the control unit, the switching means can connect the electrical stimulation circuit to the optimal pair of electrodes that correspond to the desired stimulation site. The control unit can determine the appropriate electrode pair based on pre-programmed stimulation protocols, user input, or real-time feedback from sensors that monitor the user's physiological responses.

In one embodiment of the present invention, the switching means can be realized as a switching matrix. The switching matrix comprises a network of interconnected switches or transistors that can be individually controlled by the control unit to selectively connect the electrical stimulation circuit to any desired pair of electrodes within the electrode arrangement.

The present invention, in a preferred embodiment, comprises an electrical stimulation circuit configured to generate and deliver low-frequency electrical impulses with a frequency between 1 Hz and 1 kHz through at least one pair of electrodes in the electrode arrangement. These low-frequency electrical impulses are sufficient to elicit nerve potentials on the surface of the skin without penetrating deeper tissues. This superficial stimulation is particularly advantageous for targeting the auricular nerves, which are located in close proximity to the skin surface.

According to another preferred embodiment, the electrode arrangement comprises multiple pairs of electrodes, each pair of electrodes being independently controllable by the electrical stimulation circuit to generate a respective high-frequency electrical field with a frequency between 1 kHz and 500 kHz, such as between 1 kHz and several 100 kHz, wherein the high-frequency electrical fields generated by the multiple pairs of electrodes in the electrode arrangement interfere with each other, creating constructive and destructive interference patterns that produce localized potentials at various depths and locations. In such an embodiment, the control unit may preferably be configured to generate stimulation parameters for each pair of electrodes in the electrode arrangement, including the frequencies, pulse widths, amplitudes, and phase relationships of the high-frequency electrical fields, based on a desired stimulation pattern, depth, and location.

In this embodiment it is possible to stimulate regions of the ear that are not in direct contact with the electrodes. In conventional transcutaneous electrical stimulation, the stimulation current is typically confined to the tissues immediately under or nearby the electrodes, limiting the reach and depth of the stimulation. However, by leveraging the interference patterns created by multiple pairs of independently controlled electrodes, the present invention can project the stimulation fields to deeper layers of tissue or to regions that are not directly accessible by the electrodes. This is achieved through the constructive interference of the high-frequency electrical fields, which can create localized potential gradients at various depths and locations within the auricular region.

Furthermore, the dynamic control over the interference patterns allows for the stimulation focus to be modulated in real-time during the therapy session. By continuously adjusting the frequencies, pulse widths, amplitudes, and phase relationships of the electrical fields generated by each pair of electrodes, the device can create "moving" or "scanning" stimulation patterns that cover a wider area or explore different regions of the ear. This dynamic stimulation approach can be particularly useful when the optimal stimulation targets are not precisely known or when the therapy aims to modulate multiple neural pathways simultaneously. The device can cycle through different interference patterns, targeting various depths and locations, to maximize the chances of engaging the relevant nerve fibers and eliciting the desired therapeutic effects.

The present invention, in a preferred embodiment, further comprises an integrated power source disposed within the first housing and/or the second housing and electrically connected to the electrical stimulation circuit. The integrated power source is configured to supply electrical energy to the electrical stimulation circuit for generating and delivering the electrical stimulation signals. Notably, the integrated power source is capable of producing an electric field and current with sufficient strength to convey an electrical pulse through the user's outer skin layer and reach a specific nerve area of or in the region of the user's first and second ears. By eliminating the need for external power supplies or cumbersome wires, the device becomes self-contained and highly mobile. Users can wear the device comfortably and discreetly, without being tethered to a stationary power source or requiring frequent battery replacements. This increased portability allows users to receive the transcutaneous auricular nerve stimulation therapy while going about their daily activities, improving treatment adherence and overall quality of life.

The present invention, in a preferred embodiment, further comprises a signal acquisition circuit electrically connected to the electrode arrangement. The signal acquisition circuit is configured to measure and record electrical impedance and potential signals from the tissue underlying the device. Additionally, a signal processing unit is electrically connected to the signal acquisition circuit and is configured to analyze the measured electrical impedance and potential signals to extract physiological information, such as heart rate, heart rate variability, and respiration rate.

This embodiment enables real-time monitoring and assessment of the user's physiological state. By measuring the electrical impedance and potential signals from the tissue underlying the device, the system can capture information about the user's cardiovascular and respiratory functions. The electrical impedance measurements, which are sensitive to changes in tissue conductivity and blood volume, can be used to detect and track the user's heart rate and respiration rate. The potential signals, such as those originating from the heart's activity, can be analyzed to determine heart rate variability, which is an important indicator of autonomic nervous system balance and overall health status.

Moreover, the real-time monitoring and analysis of physiological signals enabled by the signal acquisition circuit and signal processing unit enables personalized and adaptive stimulation therapy. The device can leverage the extracted physiological information to optimize and tailor the stimulation parameters to the individual user's needs and responses. For example, the system may adjust the stimulation intensity, frequency, pulse width, or timing based on the user's heart rate or respiration rate, in order to maximize the therapeutic effects and minimize any potential side effects. The device can also implement closed-loop control algorithms that dynamically modulate the stimulation parameters in response to changes in the user's physiological state. Furthermore, the integration of physiological monitoring capabilities within the stimulation device enables the system to provide real-time feedback and alerts to the user or healthcare provider. For instance, if the device detects abnormal or concerning patterns in the user's heart rate or respiration, it can generate audible or visual alarms to prompt immediate attention or intervention.

Preferably, the control unit may be configured to receive the physiological information from the signal processing unit and use the physiological information to adapt the stimulation parameters of the electrical stimulation circuit based on the user's current physiological state and/or response to the electrical stimulation.

Furthermore, long-term tracking and analysis of the user's physiological data may be performed. The device can store the measured electrical impedance and potential signals, along with the extracted physiological parameters, in an internal memory or transmit them to an external storage or computing platform. This longitudinal data collection enables the system to establish baseline values for the user's vital signs and physiological state and track their progress over the course of the stimulation therapy. By analyzing trends and patterns in the physiological data, the device can provide valuable insights into the user's health status, treatment response, and overall well-being. This information can be used by healthcare providers to make informed decisions about the user's care plan, such as adjusting the stimulation protocol, prescribing additional interventions, or monitoring for potential complications. The long-term physiological data can also be aggregated and analyzed across multiple users to identify population-level trends, optimize treatment protocols, and advance the understanding of the mechanisms underlying transcutaneous auricular nerve stimulation.

In a preferred embodiment, the device of the present invention further comprises an electrical impedance tomography (EIT) module configured to perform local EIT measurements on the tissue of and in proximity to the user's first and second ears. The EIT module includes multiple pairs of electrodes in the electrode arrangement, which are configured to inject high-frequency electrical currents and measure resulting voltages. An EIT image reconstruction unit is configured to process the measured voltages and generates a tomographic image of the electrical impedance distribution within the tissue. Additionally, an anatomical structure estimation unit is configured to analyze the tomographic image generated by the EIT image reconstruction unit and estimate the anatomical structure around the user's ear, such as the locations of specific nerve fibers or blood vessels. The control unit is preferably configured to receive the estimated anatomical structure from the anatomical structure estimation unit, adjust the stimulation parameters for each pair of electrodes in the electrode arrangements, including the frequencies, amplitudes, and phase relationships of the high-frequency electrical fields, based on the estimated anatomical structure, and optimize the stimulation parameters to target specific areas around or within the user's ears. Preferably, the electrodes coupled to the stimulation circuit may be used as the electrodes of the EIT module.

In this way, a high-resolution, real-time visualization of the electrical impedance distribution within the tissue of and surrounding the user's ears may be obtained. This impedance map provides valuable information about the underlying anatomical structures, as different tissues and physiological conditions exhibit distinct impedance characteristics. For example, nerve fibers, blood vessels, and muscle tissue have different electrical properties compared to the surrounding soft tissues. By identifying these impedance variations, the EIT module enables the device to identify and localize the specific anatomical targets for stimulation.

This personalized anatomical modeling allows the device to adapt the stimulation parameters to the unique characteristics of each user's ear. The control unit receives the estimated anatomical structure from the estimation unit and may preferably adjust the stimulation parameters for each pair of electrodes accordingly. For example, the control unit may optimize the frequencies, pulse widths, amplitudes, and phase relationships of the high-frequency electrical fields to create constructive interference patterns that selectively target the desired nerve fibers while minimizing the stimulation of surrounding tissues.

Another effect of the EIT-based anatomical structure estimation and adaptive stimulation is the potential for real-time monitoring and adjustment of the therapy. The EIT module can continuously acquire impedance measurements during the stimulation sessions, providing dynamic feedback on the tissue's response to the applied electrical fields. The anatomical structure estimation unit can process these real-time impedance images to track any changes in the tissue properties or anatomical configurations over time. For example, if the first and/or second stimulation assembly's position relative to the user's ears or the contact with the electrodes slightly shifts during the session, the estimation unit can detect these changes and update the anatomical model accordingly. The control unit can then adjust the stimulation parameters in real-time to maintain optimal targeting of the desired nerve fibers.

Preferably, the device of the present invention may comprise at least one sensor selected from the group consisting of an electrocardiogram (ECG) sensor, an electroencephalogram (EEG) sensor, a photoplethysmography (PPG) sensor, an electromyography sensor (EMG), an electric skin conductance (ESC) sensor, an electromyography (EMG) sensor, an electrical field plethysmography (EFPG) sensor and an inertial measurement unit (IMU) sensor. Each of these sensors provides information that can be used to monitor the user's physiological state, assess the effectiveness of the stimulation therapy, or adapt the stimulation parameters in real-time.

The ECG sensor is designed to measure the electrical activity of the user's heart, providing insights into the cardiac function and potential abnormalities. The ECG sensor typically comprises a set of electrodes that are placed on the user's skin, to detect the electrical signals generated by the heart. Preferably, the electrodes of the electrode arrangement of the first and/or second stimulation assembly be used as said electrodes. The sensor may be connected to the necessary circuitry for signal conditioning, amplification, and digitization, as well as algorithms for heart rate detection and arrhythmia analysis. The ECG data can be used to monitor the user's cardiac response to the stimulation therapy including changes in heart rate variability used to detect changes in autonomic nervous system function, detect any adverse effects, or trigger stimulation based on specific cardiac events.

The EEG sensor is designed to measure the electrical activity of the user's brain, providing valuable information about the neural state and potential abnormalities. The EEG sensor typically comprises a set of electrodes that are placed on the user's scalp, to detect the electrical signals generated by the brain. Preferably, the electrodes of the electrode arrangement of the first and/or second stimulation assembly be used as said electrodes. The sensor may be connected to the necessary circuitry for signal conditioning, amplification, and digitization, as well as algorithms for spectral analysis, event detection, and artifact removal. The EEG data can be used to monitor the user's neural response to the stimulation therapy, assess the level of arousal or relaxation, or trigger stimulation based on specific brain wave patterns.

The PPG sensor is designed to measure the user's blood volume changes, providing information about the cardiovascular function and potential abnormalities. The PPG sensor typically comprises a light source, such as a LED, and a photodetector, which are placed in close proximity to the user's skin, such as the earlobe, or other suitable location. The sensor module may be connected to the necessary circuitry for light emission, detection, and signal processing, as well as algorithms for heart rate extraction, blood oxygen saturation estimation, and respiratory rate monitoring. The PPG data can be used to monitor the user's cardiovascular response to the stimulation therapy, detect any adverse effects, or trigger stimulation based on specific physiological parameters.

The EMG sensor is designed to measure the electrical activity of specific muscle groups of the user, providing insights into the muscle tone and contraction pattern of muscles and potential abnormalities. The EMG sensor typically comprises a set of electrodes that are placed on the user's skin, to detect the electrical signals generated by the muscle contraction. Preferably, the electrodes of the electrode arrangement of the first and/or second stimulation assembly be used as said electrodes. The sensor may be connected to the necessary circuitry for signal conditioning, amplification, and digitization, as well as algorithms for twitch detection an analysis of the muscle tone. The EMG data can be used to monitor the user's muscle tone and contraction changes in response to the stimulation therapy including changes in contraction patterns, state of muscle relaxation, or trigger stimulation based on specific myographic events.

The ESC or galvanic skin resistance sensor is designed to measure the user's change in skin conductance due to perspiration, providing information on autonomic function and stress level of the patient. The ESC sensor typically comprises a current source and measures changes in skin resistance via electrodes, which are placed in close proximity to the user's skin. Preferably, the electrodes of the electrode arrangement of the first and/or second stimulation assembly be used as said electrodes. The sensor may be connected to the necessary circuitry for current generation, voltage detection, and signal processing, as well as algorithms for detection of changes in stress level and autonomic function. The ESC data can be used to monitor the user's autonomic function response to the stimulation therapy, detect any adverse effects, or trigger stimulation based on specific physiological parameters.

The IMU sensor is designed to measure the user's motion and orientation, providing information about the physical activity and posture. The IMU sensor typically comprises a combination of accelerometers, gyroscopes, and magnetometers, which are integrated into the sensor module. The sensor can detect linear acceleration, angular velocity, and magnetic field changes, allowing for the tracking of the user's movements and orientation in three-dimensional space. The IMU data can be used to monitor the user's physical activity levels, or other abnormal movements, or adapt the stimulation parameters based on the user's posture or motion. An IMU sensor may also be used to detect specific muscle contractions, cardiac or respiration events depending on the position of the sensor on the body of the patient.

The present invention, in a preferred embodiment, includes an acoustic transducer arranged in each of the first housing and the second housing. These acoustic transducers are capable of converting electrical signals into audible vibrations, allowing for concurrent sound delivery alongside the transcutaneous auricular nerve stimulation. The acoustic transducers is capable of generating sound waves in the audible frequency range of preferably 50-16,000 Hz. Integrating acoustic transducers into the first and second housings allows to create an immersive and multisensory experience for the user. This combination of sound and electrical stimulation can have a synergistic effect, potentially enhancing the overall effectiveness and impact of the therapy. For example, in applications related to relaxation or stress reduction, the device can play soothing sounds or music while simultaneously applying electrical stimulation to the auricular nerves. Applications in tinnitus patients may be supported by a combined electrical and acoustic stimulation.

Moreover, the integration of acoustic transducers into the device housings enables the precise synchronization and coordination of sound delivery with the electrical stimulation patterns. The control unit can generate electrical signals that drive both the stimulation circuitry and the acoustic transducers in a coordinated manner. This allows for the precise timing and alignment of auditory and electrical stimuli, creating a coherent and optimized sensory experience. For example, the device can deliver specific sound frequencies or rhythms that are matched to the stimulation frequencies, potentially amplifying the neuromodulatory effects. The control unit can also adjust the volume, pitch, and other acoustic parameters dynamically based on the stimulation intensity or the user's physiological responses, ensuring an adaptive and personalized experience.

Another technical effect of the integrated acoustic transducers is the potential for using sound as a feedback or guidance mechanism during the stimulation therapy. The device can generate specific audio cues or prompts to guide the user through the stimulation session, providing instructions, reminders, or progress updates. For example, the device may play voice prompts to indicate the start or end of a stimulation cycle, or to notify the user when to adjust the device position or settings. The acoustic feedback can also be used to convey information about the stimulation parameters, such as the current intensity level or the remaining session duration, allowing the user to monitor and control the therapy easily. Additionally, the device can incorporate sound-based biofeedback mechanisms, where the acoustic output is modulated in real-time based on the user's physiological signals, such as heart rate or brain activity. This auditory feedback can help the user to self-regulate and optimize their physiological state during the stimulation session, promoting a more engaging and interactive therapeutic experience.

In a further preferred embodiment, the present invention optionally incorporates additional sensory inputs, such as vibration, in the area of the earphones. This may include a skin contacting element that is excited to mechanical vibrations of, e.g. 10-200 Hz, and is in direct contact with the user's skin. This multisensory stimulation approach combines transcutaneous auricular nerve stimulation with other sensory modalities, creating a more immersive and engaging experience for the user. By integrating vibrotactile feedback into the earphones, the device can provide tactile cues or patterns that complement the electrical stimulation and audio signals.

The transcutaneous auricular nerve stimulation device of the invention may be used for several therapeutic applications that address a wide range of neurological and physiological conditions.

Tinnitus relief: Transcutaneous auricular nerve stimulation delivered through over-ear headphones and optionally combined with acoustic stimuli can provide an effective and convenient solution for managing tinnitus symptoms, offering users relief from persistent ringing or buzzing sounds in their ears.

Pain management: Transcutaneous electrical nerve stimulation can be used to alleviate chronic pain in different body regions; over-ear headphones with integrated stimulation multi-electrode arrays can be used for managing head and neck pain, including migraines and tension headaches, back pain, post-surgery acute pain, inflammatory diseases associated with pain, or others.

Neurorehabilitation: The invention may help restore or improve physical and mental function following brain injury, stroke, or other neurological conditions by directly modulating the activity of damaged or dysfunctional neural pathways.

Other neurological and psychiatric conditions include PTSD, addiction or depression.

In the following, the invention will be described with reference to exemplary embodiments illustrated in the drawings. Therein. Fig. 1 shows an over-ear stimulator having 16 electrodes and magnetically connected ear cushions, Fig. 2 shows an over-ear stimulator having 16 electrodes and bayonet connected ear cushions, Fig. 3 shows an electrode array of a stimulation assembly with two exemplary configurations of active stimulation electrodes, Fig. 4 shows an electrode array of a stimulation assembly with an exemplary electrode configuration used for high frequency interference stimulation, and Fig. 5 illustrates a block diagram of a stimulation device of the invention.

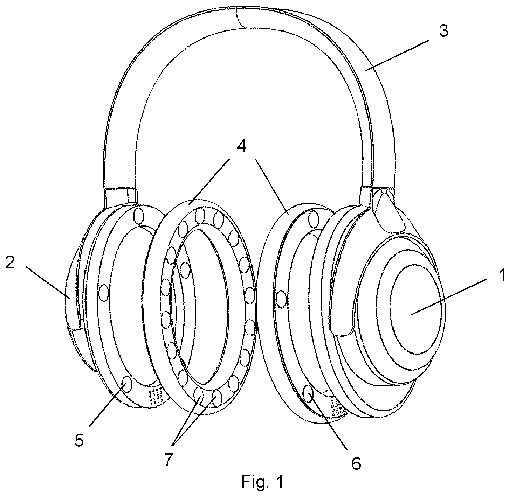

Fig. 1 illustrates a device for transcutaneous auricular nerve stimulation, comprising a first stimulation assembly 1 in the shape of an earcup of an over-ear headphone and a second stimulation assembly 2 in the shape of an earcup. Further, the device comprises a holding structure 3 designed as a headband that interconnects the first stimulation assembly 1 and the second stimulation assembly 2.

The first and the second stimulation assemblies 1,2 each comprise a resilient pad 4 configured to contact a skin surface surrounding the user's first and second ear, respectively, and having electrodes 7 embedded into or arranged on the resilient pad 4. The resilient pad 4 is removably attached to a base part of the first and the second stimulation assemblies 1,2, respectively, via a magnetic coupling mechanism comprising magnets 5 and 6.

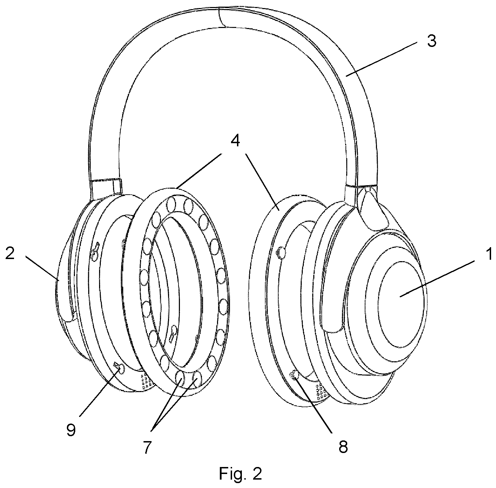

The embodiment of Fig. 2 largely corresponds to the embodiment of Fig. 1, except that a bayonet-type coupling mechanism is provided instead of a magnetic coupling mechanism. As shown in Fig. 2, the bayonet-type coupling mechanism comprises bayonet pins 8 and bayonet slots 9.

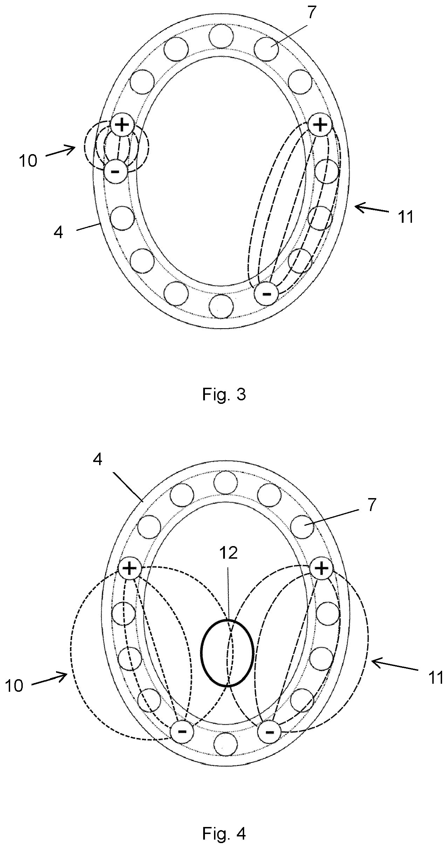

Fig. 3 illustrates an electrode arrangement on the resilient pad 4 comprising a plurality of electrodes 7 spaced apart in the circumferential direction of the skin-contacting region of the resilient pad 4. The electrode arrangement comprises a first pair 10 of electrodes 7 and a second pair 11 of electrodes 7. Each pair 10,11 of electrodes 7 is independently controllable by the electrical stimulation circuit to generate a respective electrical field, as illustrated in Fig. 3.

In Fig. 4 the first pair 10 and the second pair 11 of electrodes 7 are controllable by the electrical stimulation circuit to generate a respective high-frequency electrical field with a frequency between 1 kHz and several 100 kHz, wherein the high-frequency electrical fields interfere with each other in the interference area 12 in order to stimulate this area in a targeted manner.

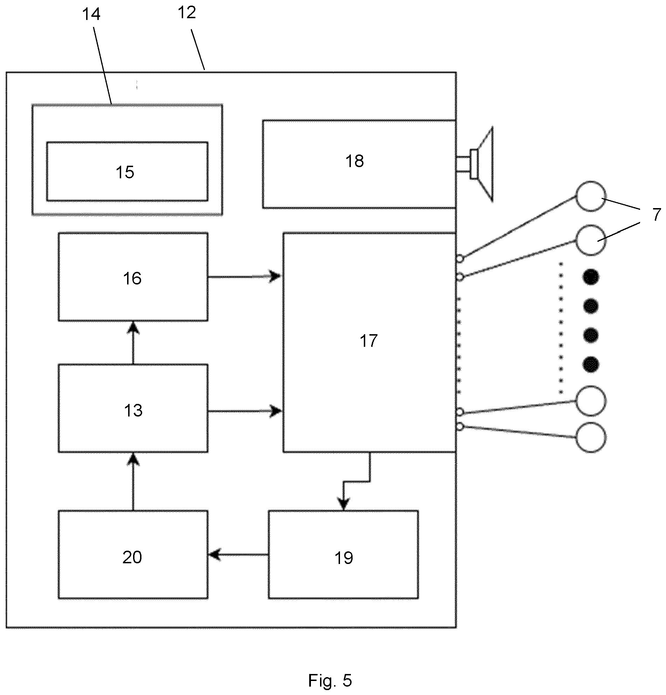

Fig. 5 illustrates a block diagram of a stimulation device 12 according to the present invention. The stimulation device 12 comprises several components that work together to deliver transcutaneous auricular nerve stimulation and acoustic stimulation to the user.

The device comprises a control unit 13, which controls and coordinates the functioning of all other components. The control unit is connected to a power supply 14, which includes a battery 15, to provide the necessary electrical energy for the device's operation.

The control unit 13 is also connected to a stimulation circuit 16, which is responsible for producing the electrical stimulation signals. The control unit 13 can adjust the stimulation parameters, such as amplitude, frequency, pulse width, and phase of the stimulation signals.

A switching unit 17 is connected between the stimulation circuit 16 and the electrodes 7. The switching unit 17 allows for the selective activation and deactivation of individual electrode pairs, enabling the device to target specific areas around the user's ear.

The electrodes 7 are arranged in an electrode arrangement, which is designed to be in contact with the user's skin around the ear. The electrode arrangement comprises multiple pairs of electrodes 7, each pair being independently controllable by the electrical stimulation circuit 16 to generate a respective electrical field.

The device also includes a signal acquisition circuit 19, which is connected to the control unit 13. The signal acquisition circuit 19 can receive biofeedback signals and have them processed in the signal processing unit 20. These signals can be used by the control unit 13 to adjust the stimulation parameters or to monitor the user's physiological state during the stimulation session.

Further, the device features an acoustic transducer 18, which is connected to the control unit 13. The acoustic transducer 18 is capable of converting electrical signals into audible vibrations, allowing for the concurrent delivery of acoustic stimulation alongside the transcutaneous auricular nerve stimulation.

The arrows in the block diagram represent the flow of information and signals between the various components of the stimulation device, illustrating the interconnected nature of the system.

## Claims

1. A device for transcutaneous auricular nerve stimulation, comprising:
a first stimulation assembly (1) comprising a first housing configured to be positioned over a user's first ear,
a second stimulation assembly (2) comprising a second housing configured to be positioned over a user's second ear,
the first and the second housing each comprising an electrode arrangement configured to contact a skin surface in proximity to the user's first and second ear, respectively,
a holding structure (3) coupled to the first stimulation assembly (1) and to the second stimulation assembly (2) and configured to be worn on or around the user's head,
a stimulation circuit (16) electrically coupled to the electrode arrangement and configured to generate electrical stimulation signals to be delivered by the electrode arrangement,
wherein the electrical stimulation signals delivered by the electrode arrangement are configured to stimulate nerves in the region of the user's first ear and the user's second ears, respectively.

2. Device according to claim 1, wherein the first and the second housing are each designed in the shape of an earcup of an over-the-ear headphone.

3. Device according to claim 1 or 2, wherein the electrode arrangement comprises at least one, preferably at least two electrodes (7) arranged in a skin-contacting region of the first and the second housing, respectively, the skin-contacting region being configured to surround the user's first and the use's second ear, respectively.

4. Device according to claim 3, wherein the electrode arrangement comprises a plurality of electrodes (7) spaced apart in the circumferential direction of the skin-contacting region.

5. Device according to claim 3 or 4, wherein the skin-contacting region comprises a resilient pad (4) configured to contact a skin surface surrounding the user's first and second ear, respectively, wherein the electrodes (7) are embedded into or arranged on the resilient pad (4).

6. Device according to claim 5, wherein the resilient pad (4) is removably attached to a base part of the first and the second housing, respectively, via a coupling mechanism, wherein the coupling mechanism preferably comprises either a magnetic coupling mechanism (5,6) or a mechanical coupling mechanism (8,9), such as a snap-fit, hook-and-loop fastener, or other releasable fastening means.

7. Device according to any one of claims 1 to 6, further comprising a control unit (13) configured to control at least one stimulation parameter of the electrical stimulation signals, such as the intensity, pulse width, frequency, and/or duration of the electrical stimulation signals.

8. Device according to claim 7, wherein the electrical stimulation circuit (16) comprises a switching means (17) electrically coupled to the plurality of electrodes (7) in the electrode arrangement, the switching means (17) configured to selectively connect the electrical stimulation circuit (16) to at least one selected pair of electrodes (7) out of the plurality of electrodes (7) in the electrode arrangement based on switching instructions received from the control unit (13).

9. Device according to any one of claims 1 to 8, wherein the electrical stimulation circuit (!6) is configured to generate and deliver low-frequency electrical impulses with a frequency between 1 Hz and 1 kHz through at least one pair of electrodes (7) in the electrode arrangement, the low-frequency electrical impulses being sufficient to elicit surface nerve potentials in the skin surface in proximity to the user's first and second ears, respectively.

10. Device according to any one of claims 1 to 9, wherein the electrode arrangement comprises multiple pairs of electrodes (7), each pair of electrodes (7) being independently controllable by the electrical stimulation circuit (16) to generate a respective high-frequency electrical field with a frequency between 1 kHz and 500 kHz, wherein the high-frequency electrical fields generated by the multiple pairs of electrodes (7) in the electrode arrangement interfere with each other, creating constructive and destructive interference patterns that produce localized potentials at various depths and locations.

11. Device according to any one of claims 1 to 10, further comprising an integrated power source (14) disposed within the first housing and/or the second housing and electrically connected to the electrical stimulation circuit (16), the integrated power source (14) being configured to supply electrical energy to the electrical stimulation circuit (16) for generating and delivering the electrical stimulation signals.

12. Device according to any one of claims 1 to 11, further comprising a signal acquisition circuit (19) electrically connected to the electrode arrangement, the signal acquisition circuit (19) being configured to measure and record electrical impedance and potential signals from the tissue underlying the device, and a signal processing unit (20) electrically connected to the signal acquisition circuit (19), the signal processing unit being configured to analyze the measured electrical impedance and potential signals to extract physiological information, such as heart rate, heart rate variability, and respiration rate.

13. Device according to claim 12, wherein the control unit (13) is configured to receive the physiological information from the signal processing unit (20) and use the physiological information to adapt the stimulation parameters of the electrical stimulation circuit (16) based on the user's current physiological state and/or response to the electrical stimulation.

14. Device according to any one of claims 7 to 13, further comprising an electrical impedance tomography (EIT) module configured to perform local EIT measurements on the tissue of and in proximity to the user's first and second ears, the EIT module comprising:
multiple pairs (10,11) of electrodes (7) in the electrode arrangement, which are configured to inject high-frequency electrical currents and measure resulting voltages,
an EIT image reconstruction unit configured to process the measured voltages and generate a tomographic image of the electrical impedance distribution within the tissue,
an anatomical structure estimation unit configured to analyze the tomographic image generated by the EIT image reconstruction unit and estimate the anatomical structure around the user's ear, such as the locations of specific nerve fibers or blood vessels,
wherein the control unit (13) is configured to receive the estimated anatomical structure from the anatomical structure estimation unit, adjust the stimulation parameters for each pair (10,11) of electrodes (7) in the electrode arrangements, including the frequencies, amplitudes, and phase relationships of the high-frequency electrical fields, based on the estimated anatomical structure, and optimize the stimulation parameters to target specific areas around the user's ears.

15. Device according to any one of claims 1 to 14, wherein an acoustic transducer (18) capable of converting electrical signals into audible vibrations is each arranged in the first housing and the second housing.

16. Device according to any one of claims 1 to 15, wherein a mechanical vibration generator is arranged in the first housing and/or the second housing.
